# EUROPEAN PATENT SPECIFICATION

(11) **EP 0 385 441 B1**
(45) Date of publication and mention of the grant of the patent: **21.04.1993**
(21) Application number: 90103908.1
(22) Date of filing: 28.02.1990
(51) Int. Cl.: C10L 1/22, C10L 1/00

(54) **Marker for petroliferous products**
Markierende Verbindung für Erdölhaltige Produkte
Marqueur pour produits pétrolifères

(30) Priority: 01.03.1989 IT 1960389
(43) Date of publication of application: 05.09.1990
(73) Proprietor: ACNA CHIMICA ORGANICA S.P.A., I-17010 Cengio (Savona) (IT)
(72) Inventor: Papa, Sisto Sergio, Dr., I-20125 Milan (IT)
(74) Representative: Barz, Peter, Dr.

(56) References cited:
- BE-A- 476 399
- FR-A- 1 206 228
- US-A- 1 423 050
- US-A- 3 434 814
- US-E- 16 937
- CHEMICAL ABSTRACTS, vol. 69, no. 4, 22 July 1968, Columbus, OH (US); S.V. ANANTAKRISHNAN et al., p. 1362, no. 14383p#
- CRC HANDBOOK OF CHEMISTRY AND PHYSICS, 60th ed., 1979-80; p. C-163#

## Description

The present invention relates to the (permanent) marking of petroliferous products, in particular diesel fuel. The marking agent employed according to the present invention comprises 1,4-dimethyl-2-nitro-benzene, or para-xylene-mononitrate, of the following formula I:

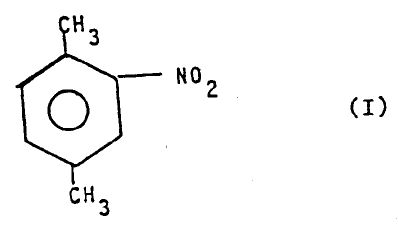

The need to mark petroliferous products, such as fuels, is due to the difference in the price which the very same petroliferous product or solvent can have, owing to the different taxation that it is subjected to according to its destination.

As a result, situations such as those leading to fiscal fraud can occur when the product is used for purposes different from those for which it was taxed.

It is in order to prevent such fraud from taking place that petroliferous products are marked with a suitable substance which is easy to identify.

The markers that can be used for this purpose must be stable under the use conditions, must be of a nature that does not affect the chemico-physical characteristics of the petroliferous product, must show sufficient solubility, preferably high enough so as to allow use thereof in the form of concentrated solutions, must be difficult or impossible to remove with physical or physico-chemical methods and must be employable in small quantities. Furthermore, they should be identifiable by means of simple, fast and sensitive detection methods.

A great number of additives and markers has been proposed and is being used, but nevertheless they either do not completely satisfy the above requirements or they show various disadvantages. Among these known marking substances, the following may be mentioned:
- organometallic compounds which, however, have the disadvantage of showing poor storage stability;
- radioactive substances which, however, require special equipment for their treatment since they are physiologically dangerous;
- primary, secondary and tertiary aromatic amines which, however, can easily be extracted with diluted acids;
- quinizarin which, however, has a poor solubility and, therefore, can only be used in powder form instead of a concentrated solution; moreover, it is easy to eliminate, for example with active carbon and adsorbent earth etc.;
- furan, diphenylamine and naphthols which, however, may easily be removed from the system; furthermore the former two are suspected of being cancerogenes.

For obvious economic reasons the use of nitroxylenes is particularly wide-spread mainly in the marking of industrial organic solvents, especially in the form of a mixture obtained from the nitration processes of technical xylene mixtures.

Nevertheless, the use of the above mixtures of nitrated xylenes is associated with some disadvantages which substantially can prevent the use thereof and/or restrict the field of use. Indeed, since the starting technical mixtures of xylenes can show considerable variations in the compositions thereof, it is evident that the composition of the nitro derivatives derived therefrom may in turn vary considerably. Therefore it is practically impossible to achieve, as necessary, a product or, alternatively, a final mixture having standard (reproducible) characteristics. Consequently, this implies a variation of the concentration in the petroliferous product according to the qualitative or quantitative characteristics of the nitro derivative mixture which in turn may result in fraud and/or law suits. Furthermore, the presence of ethylbenzene (suspected of causing cancer in man) in an amount of 10 to 15% of the starting xylene mixture also complicates the composition of the mixture.

On the other hand, the possible use of ortho-xylene or metaxylene as the substrates to be nitrated results, as it is known, in the formation of isomers and/or polynitrated compounds during the nitration process, due to the possible variable electron distribution in ortho-xylene or meta-xylene.

This results in difficulties and/or doubts regarding the reproducibility and the analytical characterization which are of fundamental importance for the application in the already established field.

Besides, the higher price and poor availability of ortho- and meta-xylene must be taken into consideration. This makes the use thereof even less desirable.

It has now surprisingly been found that the product of the mononitration of para-xylene, i.e., 1,4-dimethyl-2-nitro-benzene having formula (I) is substantially unique as far as the structure thereof is concerned and therefore it does not require purification after its synthesis (which takes place according to known nitration methods). Consequently, its preparation is doubtlessly reproducible, assuring a corresponding reliability of the standardized dosage. Finally, the concentration of 1,4-dimethyl-2-nitro-benzene is easy to determine without any difficulties and/or analytical uncertainties.

Objects of the invention, therefore, are the use of 1,4-dimethyl-2-nitro-benzene for the marking of petroliferous products, and the correspondingly marked petroliferous products.

The present marker is especially suitable for the marking of diesel fuel.

The quantities of para-xylene-mononitrate (I) to be used as marker may vary within a wide range, the minimum quantity required depending merely on the sensitivity of the employed method of determination.

By using a simple and fast determination assay, which will be described below, it is possible to use concentrations of the order of 20 to 100 ppm with respect to the product to be marked.

Generally a higher amount is added, for example from 0.5 to 5% by weight, so that if the marked product would be diluted up to 100 to 200 times its initial volume for fraud purposes, the above assay would still afford a positive result.

The use of para-xylene-mononitrate (I) as marking substance according to the present invention affords numerous advantages, some of which will be discussed in the following:
Para-xylene-mononitrate (I) is a stable substance which does not influence the characteristics of the substrate to be marked.
Owing to its chemical structure, it can practically be excluded that it is naturally present in the products to be marked.

Compound (I) is difficult to eliminate from its solutions in petroliferous products, e.g., diesel fuel, for example by means of a treatment with acids or alkalis or by means of adsorption with charcoal, alumina, silica, or bleaching clays, etc.

Furthermore, it is practically colourless and therefore its presence in petroliferous products can be detected only by means of a specific assay.

Its detection may be conducted by means of a simple assay which is devoid of interferences and has a remarkable sensitivity, of the order of 20 to 30 ppm, resulting in the further advantage that it is possible to use compound (I) as marker in extremely small quantities.

As mentioned above, compound (I) also is easy to prepare, has a reasonable price and is readily available on the market.

Para-xylene-mononitrate (I) can be put on the market and added in the form of a liquid since it is readily and completely miscible with petroliferous products.

The simplest identification method, briefly mentioned above, does not require any complicated instruments and can be carried out rapidly even by untrained personnel.

Said method consists in detecting marker (I) in petroliferous liquids and especially in diesel fuel, by means of thin-layer chromatography (TLC) for which the following equipment is required:
- chromatographic glass plates (10 x 10) coated with silica gel;
- a microsyringe or an analogous apparatus capable of releasing the tested sample in predetermined quantities;
- a chromatographic cylinder (diameter = about 12 cm);
- recommended eluents:
   a) carbon tetrachloride
   b) a toluene-ethyl acetate mixture (90:10 by volume);
   c) a hexane-acetone mixture (98:2 by volume).

For a qualitative and semi-quantitative assay the following equipment may be used:
1) a lamp for the reading of chromatographic plates with a wavelength in the U.V. spectrum (this detection method is employable up to a concentration of 0.01% by weight of the marker);
2) reagents: (5% TiCl₃ solution, NaNO₂ and HCl in solution for the formation of nitrous vapours, sulfamic acid, 1% N-(1-naphthalene)-ethylenediamine bichlorohydrate solution in dimethylformamide, 5% sodium carbonate solution for the reduction of para-xylene-mononitrate (I) on chromatographic plates and the colorimetric development by means of diazo-coupling (sensitivity of this method: 2 ppm).

Naturally, besides the chromatographic analysis, chemical or more sensitive chemico-physical methods of identification can be used. These methods are, however, also more complex and slower, but generally make it possible to determine the presence of even traces of nitroxylene.

The following examples are given for merely illustrative purposes.

### EXAMPLE 1

1 g of 1,4-dimethyl-2-nitro-benzene (I) is dissolved in 100 ml of diesel fuel.
A concentration of 1% (w/v) is obtained without giving rise to a characteristic colour. 2 µl of diesel fuel marked in this way are placed on a TLC plate (silica gel, 10 x 10) along with a comparative sample of 2 µl of non-treated diesel fuel.
The TLC plate is subjected to elution with carbon tetrachloride for approximately two hours.

Thereafter, the dried plate is exposed to U.V. rays emitted by a lamp used for the reading of chromatographic plates.

In addition to components already present in non-treated diesel fuel, a component at R_{f}=0.4-0.5 can be detected which corresponds to the 1,4-dimethyl-2-nitrobenzene used as marker.

In the unmarked diesel fuel the band at R_{f} = 0.4 to 0.5 is absent.

### EXAMPLE 2

1 ml of marked diesel fuel of example 1 (containing 1% of marker (I)) is mixed with 99 ml of unmarked diesel fuel. 100 ml at 0.01% of marker (I) are obtained. A chromatographic analysis is carried out as in example 1 by placing 10 µl of said 0.01% solution on a chromatographic plate.
Once again a component at R_{f} = 0.4 - 0.5 (corresponding to 1,4-dimethyl-2-nitro-benzene) can be detected.

### EXAMPLE 3

20 ml of marked diesel fuel from example 2 at 0.01% (w/v) of marker (I) are mixed with 80 ml of unmarked diesel fuel.
100 ml at 0.002% of marker (I) are obtained.
10 µl of diesel fuel thus marked are placed on a TLC plate (silica gel, 10 x 10 cm).
The TLC plate is eluted with eluent (b), toluene-ethyl acetate (90:10 by volume) for about one hour.
Thereafter, the dried plate is sprayed with a 5% TiCl₃ solution and then put in an oven at 90° - 100°C for about 10 minutes.
After cooling, the TLC plate is immersed in nitrous vapours for 1-2 minutes. Then the following solutions are sprayed thereon in the given order:
- 5% sulfamic acid solution;
- 1% N-(1-naphthyl)ethylenediamine bichlorohydrate solution in dimethylformamide;
- 5% sodium carbonate solution.

A purple band develops at R_{f} = 0.4 - 0.5. In the unmarked diesel fuel this band is absent.

### EXAMPLE 4 (comparison)

The procedure of example 1 was followed, replacing 1,4-dimethyl-2-nitro-benzene (I) by 1 g of a mixture of nitroxylene isomers. The reading of the chromatographic plate showed that in addition to the components already present in the non-treated diesel fuel at least 4 other components were present at R_{f} = 0.5 - 0.45 - 0.40 - 0.35, corresponding to the various nitroxylene isomers.

Said components can be distinguished by their different intensities which depend on the composition of the starting nitroxylenes.

Also in order to simulate possible fraud, 1 ml of diesel fuel marked with a mixture of nitroxylene isomers at 1% was diluted with 99 ml of unmarked diesel fuel, thereby obtaining a diesel fuel solution marked with 0.01% of nitroxylenes.

The chromatographic analysis described in example 2 was carried out, placing 10 µl of the 0.01% solution on the TLC plate. Only 2 of the 4 components could be detected. The uncertainty which arose from the evaluation of the results regarding the diesel fuel under examination is evident.

In the unmarked diesel fuel the bands at R_{f} = 0.5 to 0.35 were absent. Similar results were obtained when repeating the procedure described in example 3.
Comparable results were obtained with the use of other petroliferous fractions such as kerosene, petrol, benzene, toluene and xylene.

## Claims

1. Use of 1,4-dimethyl-2-nitro-benzene for the marking of petroliferous products, particularly diesel fuel.

2. Use according to claim 1,wherein 1,4-dimethyl-2-nitrobenzene is added to the petroliferous products in quantities of from 0.5% to 5% by weight.

3. Petroliferous product, especially diesel fuel, marked with 1,4-dimethyl-2-nitro-benzene.

## Patentansprüche

1. Verwendung von 1,4-Dimethyl-2-nitrobenzol zur Markierung von erdölhaltigen Produkten, insbesondere Dieselbrennstoff.

2. Verwendung nach Anspruch 1, worin 1,4-Dimethyl-2-nitrobenzol den erdölhaltigen Produkten in Mengen von 0,5 bis 5 Gew.-% zugesetzt wird.

3. Erdölhaltiges Produkt, insbesondere Dieselbrennstoff, markiert mit 1,4-Dimethyl-2-nitrobenzol.

## Revendications

1. Utilisation de 1,4-diméthyl-2-nitrobenzène pour le marquage de produits pétrolifères, en particulier le carburant diesel.

2. Utilisation suivant la revendication 1, dans laquelle du 1,4-diméthyl-2-nitrobenzène est ajouté au produit pétrolifère en des quantités de 0,5 à 5% en poids.

3. Produit pétrolifère, en particulier un carburant diesel, marqué avec du 1,4-diméthyl-2-nitrobenzène.
